# EUROPEAN PATENT SPECIFICATION

(11) **EP 2 478 881 B1**
(45) Date of publication and mention of the grant of the patent: **26.10.2016**
(21) Application number: 10817293.3
(22) Date of filing: 17.09.2010
(51) Int. Cl.: A61F 13/15, A61F 13/472, A61F 13/53, A61F 13/539, A61F 13/47, A61F 13/475, A61F 13/511

(54) **ABSORPTIVE ARTICLE**
SAUGFÄHIGER ARTIKEL
ARTICLE ABSORBANT

(30) Priority: 18.09.2009 JP 2009218184
(43) Date of publication of application: 25.07.2012
(73) Proprietor: Unicharm Corporation, Ehime 799-0111 (JP)
(72) Inventor: HARADA, Hiroyuki, Kanonji-shi Kagawa 769-1602 (JP); MARUYAMA, Takashi, Kanonji-shi Kagawa 769-1602 (JP); NOGUCHI, Jyunichi, Kanonji-shi Kagawa 769-1602 (JP)
(74) Representative: Carr, Anne Marie
(86) International application number: PCT/JP2010/066229
(87) International publication number: WO 2011/034180

(56) References cited:
- WO-A1-2011/126143
- JP-A- H08 510 150
- JP-A- 2001 178 775
- JP-A- 2002 531 172
- JP-A- 2006 326 299
- JP-A- 2009 165 862
- US-A1- 2006 116 653
- US-A1- 2006 276 767

## Description

### [Technical Field]

The present invention relates to an absorbent article including a compression region in which a density of an absorber is increased by a compression process.

### [Background Art]

In order to prevent leakage of fluid or improve a fit feeling of absorbent articles such as sanitary napkins, it has been proposed to provide the absorbent article with a channel in an absorbent surface by a compression process (see Patent Document 1).

Patent Document 1 discloses the channel formed so as to include a high compression region in which an absorber has an increased density and a low compression region in which a density is lower than that of the high compression region, by being compressed by a compression roller having a projected portion. The high compression region is formed within the low compression region.

Generally, it has been known that the absorber exhibits higher absorbability in a high-density portion compared with a low-density portion. Thus, in the absorbent article described in Patent Document 1, fluid is absorbed by the absorbent article from a non-compression portion through the low compression region to the high compression region.

Since the high compression region is surrounded by the low compression region in the absorbent article described in Patent Document 1, in a case where the low compression region comes in contact with fluid of an amount in excess of its absorbent capacity, the fluid may undesirably spread across the channel into the absorbent surface during the period from when the fluid is shifted from the low compression region to the high compression region to when the low compression region regains an absorbing ability. This delay in absorption results in leakage of the fluid to the exterior of the absorbent article.

In view of this, it is assumed that absorption of the fluid is accelerated by increasing an area of the high compression region. However, direct formation of the high compression region on the absorbent surface without the low compression region causes an increased stress exerted on a topsheet around the high compression region, thereby leading to a problem that the absorbent surface easily tears along a boundary between a non-compression region and the high-compression region. US 2006/0116653 and US 2006/0276767 disclose the features of the preamble of claim 1.

### [Prior Art Document]

### [Patent Document]

Patent Document 1: Japanese Patent Application Publication No. 2000-14701 (Fig. 5 for example)

### [Summary of Invention]

An absorbent article (absorbent article 1) of first aspect includes: a topsheet (topsheet 10) of liquid-permeable having a skin contact surface brought in contact with a skin of a wearer; a backsheet (backsheet 20) of liquid-impermeable; and an absorber (absorber 30) disposed between the topsheet and the backsheet. The absorbent article is provided with a compression region (low compression region 53) in a channel form at a side of the skin contact portion, the compression region having an increased density with respect to at least the topsheet and the absorber by a compression process. The compression region is provided with a high compression region (high compression regions 54A, 54B) having a further increased density. The high compression region has a contact portion (contact portions 54a, 54b) in point-contact with a non-compression region not subject to compression, in a plan view of the absorbent article. The invention provides an absorbent article as defined in the appended claims.

### [Brief Description of Drawings]

Fig. 1 is a plan view showing an absorbent article according to an embodiment of the present invention as seen from a skin contact surface side of a user.
Fig. 2 is a perspective view including a cross section taken along the line X-X of the absorbent article in Fig. 1.
Fig. 3(a) is an expanded view of a compression region of the absorbent article in accordance with the invention at the skin contact surface side and Fig. 3(b) is an expanded view of a cross section of the compression region.
Fig. 4(a) is an expanded view illustrating another pattern of the compression region of the absorbent article in accordance with the invention at the skin contact surface side and Fig. 4(b) is an expanded view of a cross section of the compression region.
Figs. 5(a) and 5(b) are expanded views each illustrating yet another pattern of the compression region of the absorbent article at the skin contact surface side.
Fig. 6 is an expanded view illustrating still another pattern of the compression region of the absorbent article at the skin contact surface side.
Fig. 7 is a schematic view illustrating an outline of a device for forming the compression region.
Fig. 8 is an expanded view showing region F7 of a roller included in the device for forming the compressing region.

### [Description of Embodiments]

An embodiment of an absorbent article according to the present invention will be described with reference to the drawings. Note that, in the following description of the drawings, the same or similar numerals denote the same or similar portions. In addition, it should be noted that the drawings are schematic and ratios of dimensions and the like are different from actual ones. Therefore, specific dimensions and the like should be determined in consideration of the following description. Moreover, as a matter of course, the drawings also include portions having different dimensional relationships and ratios from each other.

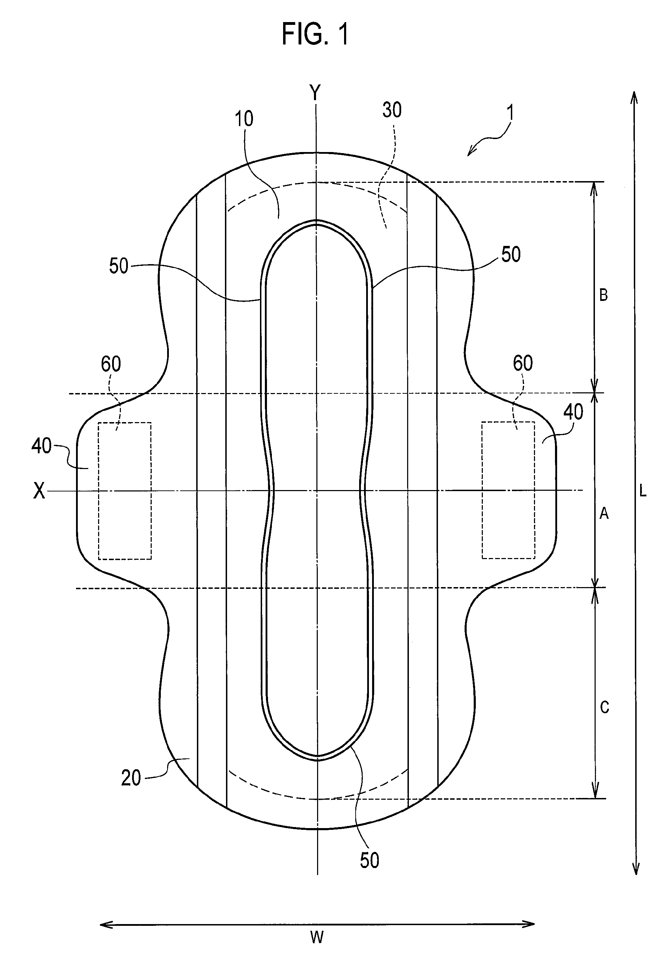

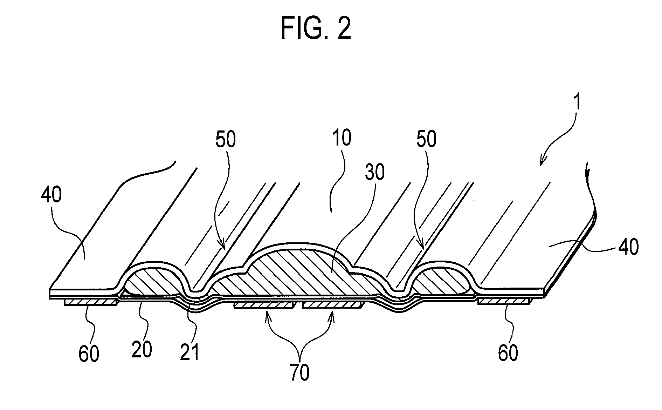

Fig. 1 is a plan view showing an absorbent article 1 according to this embodiment as seen from a topsheet side (a skin contact surface side of a user). Fig. 2 is a perspective view including a cross section taken along the line X-X of the absorbent article 1 in Fig. 1.

The absorbent article 1 is made up of a liquid-permeable topsheet 10, a liquid-impermeable backsheet 20, an absorber 30 disposed between the topsheet 10 and the backsheet 20, and wing portions 40 made from nonwoven fabric, projected laterally from both end sides of the absorbent article 1. The absorbent article 1 has a central region A in a longitudinal direction L, as well as an anterior region B and a posterior region C positioned outside the central region A in the longitudinal direction L.

The topsheet 10 is nonwoven fabric in this embodiment. The topsheet 10 is not particularly limited in raw material as long it is made from sheet-shaped material with a liquid-permeable structure, such as woven fabric or a perforated plastic sheet. Both natural fiber and chemical fiber may be used as material of woven fabric or nonwoven fabric. In this embodiment, only the topsheet 10 is disposed at the skin contact surface side of the absorber 30.

Examples of natural fiber include cellulose such as ground pulp or cotton. Examples of chemical fiber include regenerated cellulose such as rayon or fabric rayon, semi-synthetic cellulose such as acetate or triacetate, thermoplastic hydrophobic chemical fiber, or thermoplastic hydrophobic chemical fiber treated by a hydrophilic treatment. Examples of thermoplastic hydrophobic chemical fiber include a mono filament such as polyethylene (PE), polypropylene (PP), polyethylene terephthalate (PET), fiber obtained by graft-polymerizing polyethylene and polypropylene, and composite fiber with, e.g., a core-clad structure.

As a web forming method of nonwoven fabric, either one of a dry type (such as a card method, a spun bond method, a melt blown method, or an air-laid method) or a wet type may be adopted. A combination of the plurality of methods from the dry type method and the wet type method may also be adopted. The web forming method further includes a thermal bonding method, a needle punch method, and a chemical bonding method. A method of forming nonwoven fabric is not limited to the aforementioned methods.

Furthermore, as the topsheet 10, spunlace formed in a sheet shape by a hydroentangling method may be used. Yet further, nonwoven fabric may also be used as the topsheet 10, such as nonwoven fabric having a patterned indented face at a top layer side or a patterned indented nonwoven fabric which is obtained by forming unevenness on nonwoven fabric by applying air at time of web formation. Formation of unevenness on the surface achieves reduction in body fluid spreading along a surface of the topsheet 10 before permeation thereto.

In this embodiment, a core wrap 21 is disposed more closely to clothing than the absorber 30. The core wrap 21 is a sheet having absorbability such as tissue and absorbs the liquid. The backsheet 20 is disposed more closely to the clothing than the core wrap 21. As the backsheet 20, for example, a film mainly made from, e.g., polyethylene or polypropylene, a perforated resin film, or a sheet obtained by bonding the perforated resin film to nonwoven fabric such as spunbond or spunlace nonwoven fabric. It is preferable that the backsheet 20 be made from material having enough flexibility to prevent a wearer from having an uncomfortable feeling at time of wearing. For example, it is preferable to use a film mainly made from low-density polyethylene (LDPE), with a weight (weight (g) per unit area, referred to as a basis weight) within a range of 15 to 30g/m².

In this embodiment, it is preferable to make the absorber 30 from any one or a combination of, e.g., pulp, chemical pulp, rayon, acetate, natural cotton, a polymer absorber, a fibrous polymer absorber, synthetic fiber, and foam, so that the absorber 30 hardly loses shape while giving little chemical stimulation. Examples of hydrophilic fiber include any one or a combination of cellulose such as ground pulp or cotton, regenerated cellulose such as rayon or fabric rayon, semi-synthetic cellulose such as acetate or triacetate, a particulate polymer, a fibrous polymer, thermoplastic hydrophobic chemical fiber, or thermoplastic hydrophobic chemical fiber treated by a hydrophilic treatment.

Among these examples, it is preferable to use ground pulp in consideration of low cost and the formability of the absorber. The absorber 30 obtained by combining hydrophilic fiber with a polymeric absorber may also be used. In this embodiment, the polymeric absorber is an absorbent hygroscopic granular polymer such as sodium acrylate copolymer.

The absorber 30 may be an air-laid sheet obtained by shaping the hydrophobic fiber or powder in a sheet form by the air-laid method. In a case of using the air-laid sheet as the absorber 30, the air-laid sheet preferably has a thickness of 0.3 to 5.0mm. Examples of the air-laid sheet include a combination of fiber and a fibrous polymer shaped in a sheet form with the use of a binder or the like. The fibrous polymer may be dispersed in a layer form in the air-laid sheet or may be one-sided in a thickness direction.

The absorber 30 may be made up of a single layer or a plurality of layers. Further, a sheet such as tissue, a cushion sheet, or a diffusion sheet may be disposed between the topsheet 10 and the absorber 30.

A pair of wing portions 40 are formed in the central region A of the absorbent article 1. The wing portions 40 are projected outwardly in a width direction of the absorbent article 1. The absorbent article 1 has fixture portions 60 on a surface of the pair of wing portions 30, on the opposite of a wear's skin side. The fixture portions 60 are to be fixed to underwear of the wearer. The fixture portions 60 are indicated by a dashed line in Fig. 1. The fixture portions 60 are provided with an adhesive member, adhesive tape, a hook-and-loop fastener, and the like. Further, adhesive members 70 in a form of a plurality of lines are applied in the longitudinal direction L onto a surface of the backsheet 20 which is brought in contact with the underwear of the wearer (not shown in Fig. 1, see Fig. 2). The pair of wing portions 40 are folded back on a crotch portion of the wear and fastened to the underwear by the adhesive members disposed on the fixture region 60.

As an adhesive member, a hot-melt adhesive member is used, which can be easily applied in a predetermined pattern. The hot-melt adhesive member is made from styrene polymer, tackifier, and plasticizer. As the styrene polymer, styrene-ethylene-butadiene-styrene block copolymer, styrene-butadiene-styrene block copolymer, styrene-isobutylene-styrene block copolymer, or the like may be used. In this embodiment, styrene-ethylene-butadiene-styrene block copolymer was used. While the adhesive member is not limited to the above examples, soft hot-melt adhesive having pressure sensitivity at room temperature may be used, which can be penetrated and adhered at room temperature into a gap between fiber and fiber of an adherend.

As shown in Figs. 1 and 2, the absorbent article 1 is provided with a compression region 50 in which the absorber 30 is compressed by a compression process. The compression region 50 has a thickness thinner than that of a region other than the compression region 50, of the absorber 30. Herein, the region other than the compression region 50 is referred to as a non-compression region 30a.

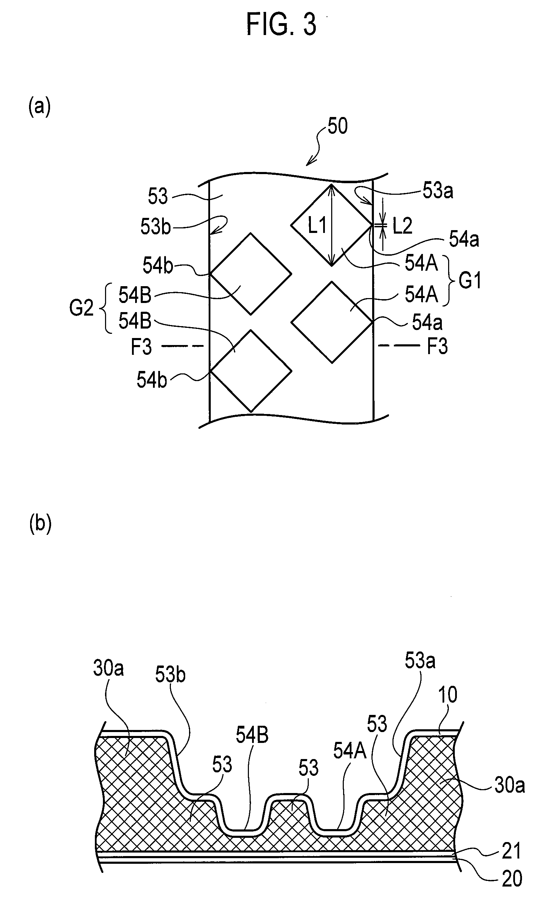

Next, the compression region 50 will be explained. Fig. 3(a) is a plan view of the compression region 50 as seen from the skin contact surface side. Fig. 3(b) is a cross section view showing a cross section of the compression region 50 taken along the line F3-F3 in Fig. 3(a).

The compression region 50 is formed in a channel shape, in which a density of at least the topsheet 10 and the absorber 30 is increased by the compression process in a plan view from the skin contact surface side. The compression region 50 is provided with high compression regions 54A, 54B in which a density is further increased. The compression region 50 is also referred to as a low compression region 53 since a density of the compression region 50, which is increase by compression, is higher than that of a non-compression region 30a but is lower than that of the high compression regions 54A, 54B.

The low compression region 53 is formed in the longitudinal direction L of the absorbent article in a plan view of the absorbent article 1. A plurality of the high compression regions 54A, 54B are formed at predetermined intervals inside the channel (low compression region 53) of the absorbent article 1 in a manner to be placed therealong.

As shown in Fig. 3(a), the high compression region 54A has a contact region 54a in contact with the non-compression region 30a in a plan view of the absorbent article. The contact portion 54a has a length L2 in an extending direction of the channel (low compression region 53). The length L2 is smaller than a length L1 of each of the high compression regions 54 in the extending direction of the channel (low compression region 53). In this embodiment, the contact portion 54a of the high compression region 54 is in point-contact with the non-compression region 30a.

The compression region 50 includes a first group G1 made up of the plurality of high compression regions 54A and a second group G2 made up of the plurality of high compression regions 54B. The high compression regions 54A making up the first group G1 have the contact portions 54a in contact with one end side 53a of the low compression region 53. That is, the first group G1 makes up the first group of high compression regions. The high compression regions 54B making up the second group G2 have contact portions 54b in contact with the other end side 53b of the low compression region 53. That is, the second group G2 makes up the second group of high compression regions.

In the example shown in Fig. 3, the first group of high compression regions 54A and the second group of high compression regions 54B are formed in a staggered configuration in the extending direction of the channel. Each of the high compression regions 54A is in a quadrilateral form in a plan view of the absorbent article 1. Each of the contact portions 54a is one vertex of the quadrilateral. Similarly, the high compression regions 54B are also quadrilateral and each of the contact portions 54b is one vertex of the quadrilateral.

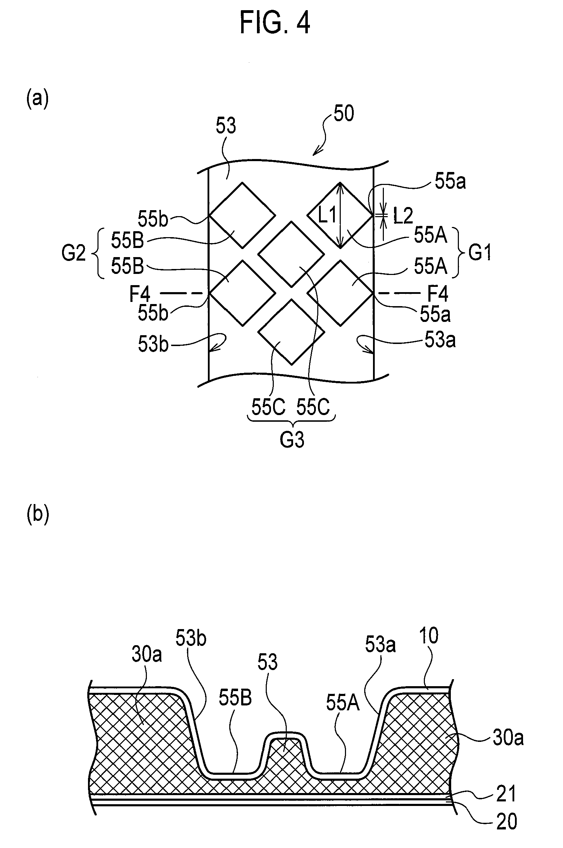

Fig. 4(a) is a plan view from the skin contact surface side, for showing another pattern of the high compression regions formed in the low compression region 53. Fig. 4(b) is a cross section view showing a cross section taken along the line F4-F4 in Fig. 4(a). In the example shown in Fig. 4, the low compression region 53 is provided with high compression regions 55A, 55B, 55C. As shown in Fig. 4, the low compression region 53 includes the first group G1 made up of the plurality of high compression regions 55A, the second group G2 made up of the plurality of high compression regions 55B, and a third group G3 made up of the plurality of high compression region 55C. The high compression regions 55A making up the first group G1 each have a contact portion 55a in contact with one end side 53a of the low compression region 53. The high compression region 55B making up the second group G2 each have a contact portion 55b in contact with the other end side 53b of the lower compression region 53. The high compression regions 55C making up the third group G3 are formed between the first ground and the second group in a width direction of the channel (low compression region 53), so as to be parallel to the extending direction of the channel (low compression region 53).

The first group of high compression regions 55A and the third group of high compression regions 55C are formed in a staggered configuration. Further, the second group of high compression regions 55B and the third group of high compression regions 55C are formed in a staggered configuration.

It is generally known that in the absorber, a region having a higher density draws in fluid more easily. As described above, the absorbent article 1 includes the low compression region 53 a density of which is increased to be higher than that of the non-compression region, and the high compression regions 54A, 54B a density of which is further increased to be higher than that of the low compression region 53. Therefore, the fluid such as body fluid is easily drawn in from the low compression region 53 to the high compression regions 54A, 54B, thereby being easily drawn in the inside of the absorber 30. Thus, the absorbent rate of the fluid is improved, so that the fluid can be prevented from being leaked to the exterior of the absorbent article 1.

In the absorbent article 1, the contact portions 54a, 54b of the high compression regions 54A, 54B are in point-contact with the non-compression region 30a not subject to compression, that is, the end sides 53a, 53b of the low compression region 53, so that the stress exerted on the topsheet 10 is small in the contact portions 54a, 54b. This prevents damages such as breakage caused on the topsheet in manufacturing steps.

A large number of high compression regions which easily draw in the fluid can be disposed in a limited area (the low compression region 53) by arranging the low compression regions and the high compression regions formed in the compression region 50 in a staggered configuration as shown in Fig. 3. This increases the amount of fluid which can be absorbed at one time. Accordingly, the absorbability of the absorbent article 1 can be improved.

In this embodiment, only the topsheet 10 is disposed at the skin side with respect to the absorber 30. The fluid which has permeated the topsheet 10 comes in direct contact with composition elements of the absorber 30, such as pulp, hydrophilic fiber, a polymer absorber, or the like, so that the absorbent rate of the fluid is accelerated.

Therefore, the absorbability of the fluid can be further improved by forming the high compression regions 54A, 54B and the low compression region 53 in the absorbent article in combination with a structure that no core wrap 21 is provided to the skin contact surface side. In this manner, leakage of the fluid can be certainly prevented.

In the above description, advantageous effects of the absorbent article provided with the high compression regions 54A, 54B shown in Fig. 3 were explained. However, even the absorbent article provided with the high compression regions 55A, 55B, 55C, shown in Fig. 4, produces similar advantageous effects. In a case of forming the compression region shown in Fig. 4 at the skin contact surface side, owing to existence of the third group G3, an area of the high compression regions is enlarged to thereby further accelerate the absorbent rate.

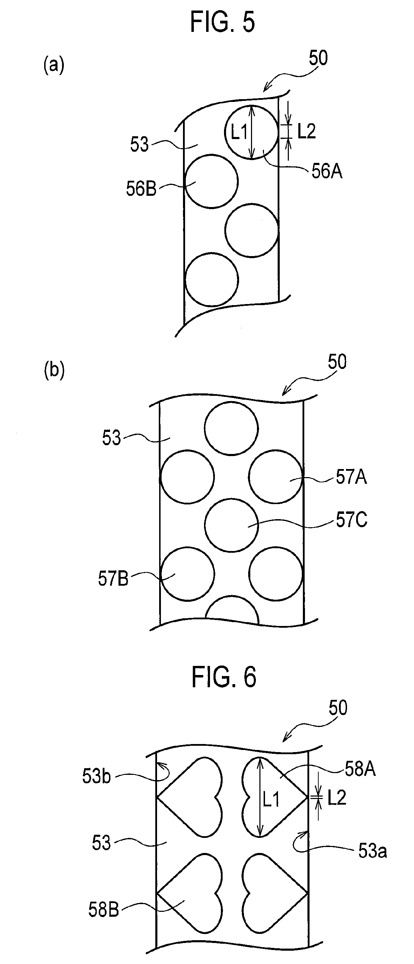

Next, another pattern of the high compression regions formed in the compression region 50 is explained. Figs. 5(a), 5(b) are plan views each illustrating another pattern of the high compression regions formed in the compression region 50 as seen from the skin contact surface side. Fig. 5(a) shows an example in which the low compression region 53 is provided with high compression regions 56A, 56B, in a substantially circular shape. Fig. 5(b) shows an example in which the low compression region 53 is provided with high compression regions 57A, 57B, 57C, in a substantially circular shape. Fig. 6 shows an example in which the low compression region 53 is provided with high compression regions 58A, 58B, in a heart shape.

In a case of the heart-shaped high compression regions 58A, 58B, it is preferable to form the compression regions 58A, 58B so that apexes 58a, 58b of the heart-shaped regions are in point-contact with the end sides 53a, 53b of the non-compression region 30a not subject to compression. In this manner, the stress exerted on the topsheet 10 can be decreased. This prevents damage to the absorbent surface.

As shown in Figs. 5 and 6, as long as the contact portion as a contact area between the high compression regions and the non-compression region has the length L2 in the extending direction of the channel (low compression region 53) smaller than the length L1 of each of the high compression regions in the extending direction of the channel, a shape of each of the high compression regions is not limited to a quadrilateral but may be shaped in a circular shape, a pattern such as a flower shape, a mark, or the like.

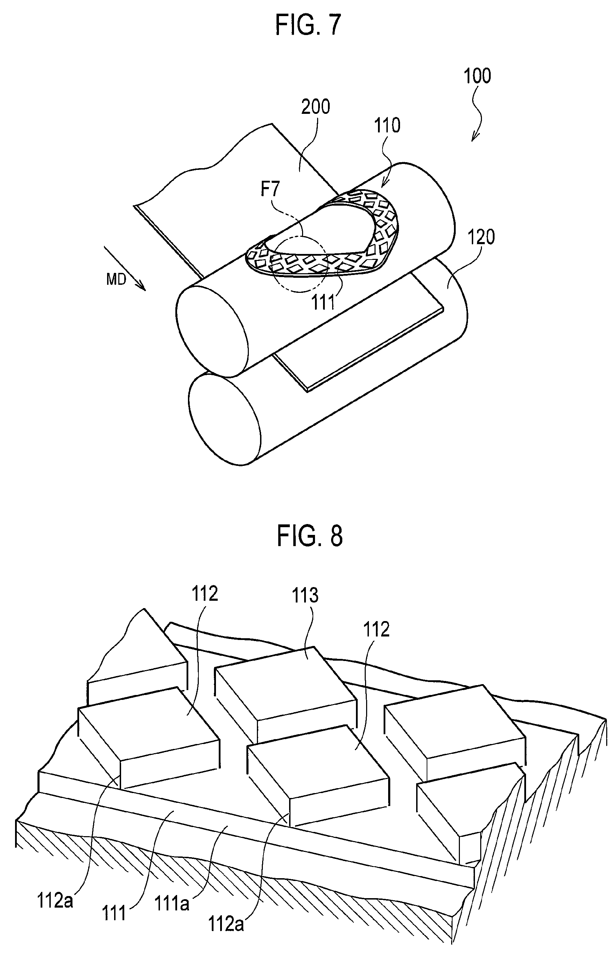

Next explained is a method of forming the low compression region and the high compression regions in the absorbent article 1 at the skin contact surface. Herein, a method of forming a pattern of the high compression regions 54A, 54B shown in Fig. 3 is explained as one example. Fig. 7 is a schematic view illustrating an outline of a device for forming the compression region 50. As shown in Fig. 7, a device 100 includes a first roller 110 and a second roller 120.

The first roller 110 is rotated in a machine direction MD along a flow direction through the manufacturing steps for the absorbent article 1 while coming in contact with a continuous body 200 containing the absorber 30 between the topsheet and the backsheet. The first roller 110 has a surface provided with a compressing portion 111 for compressing the continuous body 200. The compressing portion 111 is projected in a direction of a normal of the first roller 110, in which a projected portion configured to form the high compression regions 54A, 54B is formed on a surface brought in contact with the continuous body 200. A surface of the second roller 120 is processed to be flat.

Fig. 8 is an expanded view of the compressing portion 111. The compression portion 111 has a surface in contact with the continuous body 200, the surface being provided with projected portions 112, 113. The projected portions 112 form the high compression regions 54A. The projected portions 113 form the high compression regions 54B. Each of the projected portions 112 has a corner portion 112a. The corner portions 112a are arranged in a row on an extended line at a side surface 111 a in the direction of the normal of the first roller 110. More specifically, each of the corner portions 112a is formed so as to correspond to the side surface 111a of the compressing portion 111.

The continuous body 200 is conveyed in the machine direction MD while being sandwiched and pressed between the first roller 110 and the second roller 120. At this time, the continuous body 200 is pushed from one surface side, against the surface of the second roller 120 by the compressing portion 111 of the first roller 110. Accordingly, the continuous body 200 is provided with the compression region corresponding to a shape of the compressing portion 111.

As shown in Fig. 8, the side surface 111 a of the compressing portion 111 formed on the surface of the first roller 110 corresponds to the corner portions 112a of the projected portions 112, so that the whole of respective projected portions 112 falls within a width of the compressing portion 111. This prevents a defect such as chipping of the corner potion 112a, thereby producing an effect that roller life is extended.

(Another embodiment) As described above, the details of the embodiment of the present invention has been exemplarily disclosed. However, it should not be understood that the description and drawings which constitute part of this disclose limit the present invention. Based on this disclosure, various alternative embodiments, examples, and operation techniques are apparent to those skilled in the art.

For example, the foregoing embodiment of the present invention described a case in which the absorbent article is a sanitary napkin. However, the absorbent article is not limited to the sanitary napkin but may be a diaper, a pantiliner, a urine-receiving pad, and the like. Further, the foregoing embodiment described that the wing portions 40 are projected laterally from the both end sides of the absorbent article 1. However, the wing portions 40 may not be formed.

The high compression region formed in the compression region 50 is not limited to shapes shown in Figs. 3 to 6. The end sides 53a, 53b, of the low compression region may not form a straight line but may form a waveform or the like.

The above embodiment described a case in which only the topsheet 10 is disposed at the skin side of the wearer. However, the core wrap 21 may be disposed between the topsheet 10 and the absorber 30.

Further, the recessed and projected portion (the high compression regions 54A, 54B and the low compression regions 53A, 53B, 53C) may be formed from a clothing contact surface side of the backsheet 20.

As described above, the present invention naturally includes various embodiments which are not described herein. Accordingly, the technical scope of the present invention should be only determined according to the subject matters recited in the scope of claims which is appropriate based on the foregoing description.

### [Industrial Applicability]

According to the present invention, provided is an absorbent article having a high compression region and a low compression region, which prevents damage to an absorbent surface in the manufacturing steps while exhibiting improved absorbability of fluid.

## Claims

1. An absorbent article (1) comprising:
a liquid-permeable topsheet (10) having a skin contact surface brought in contact with a skin of a wearer;
a liquid-impermeable backsheet (20); and
an absorber (30) disposed between the topsheet and the backsheet, wherein:
the absorber is provided with a compression region (50) in a channel form at a side of the skin contact surface, the compression region having an increased density with respect to at least the topsheet and the absorber by a compression process;
the compression region is provided with a high compression region (54A, 54B, 55A, 55B) having a further increased density; **characterised in that**
the high compression region has a contact portion (54a, 54b, 55a, 55b) in point-contact with a non-compression region (30a) not subject to compression, in a plan view of the absorbent article;
wherein
the high compression region is shaped in a quadrilateral in the plan view of the absorbent article; and
the contact portion is one vertex of the quadrilateral;
wherein:
in the plan view of the absorbent article,
the compression region (50) is formed in a longitudinal direction (L) of the absorbent article;
a plurality of high compression regions (54A, 54B, 55A, 55B) are formed at predetermined intervals in the longitudinal direction of the absorbent article;
the absorbent article further includes:
a first group (G1) of the high compression regions having the contact portions (54a, 55a) in point-contact with one end side of the compression region in the channel form; and
a second group (G2) of the high compression regions having the contact portions (54b, 55b) in point-contact with the other end side of the compression region in the channel form.

2. The absorbent article according to claim 1, wherein:
the first group (G1) of the high compression regions (54A) and the second group (G2) of the high compression regions (54B) are arranged in a staggered configuration.

3. The absorbent article according to claim 1, wherein:
the absorbent article further includes:
a third group (G3) of high compression regions (55C) formed between the first group (G1) of the high compression regions (55A) and the second group (G2) of the high compression regions (55B); wherein the first group (G1) of the high compression regions (55A) and the third group (G3) of the high compression regions (55C) are arranged in a staggered configuration; and
the second group (G2) of the high compression regions (55B) and the third group (G3) of the high compression regions (55C) are arranged in a staggered configuration.

4. The absorbent article according to claim 1 further comprising:
an absorbent sheet (21) for absorbing fluid, wherein:
the absorbent sheet is disposed between the absorber (30) and the backsheet (20); and
only the topsheet (10) is disposed at the side of the skin contact surface of the absorber (20).

## Patentansprüche

1. Ein absorbierender Artikel (1), der Folgendes beinhaltet:
eine flüssigkeitsdurchlässige Oberlage (10), die eine mit einer Haut eines Trägers in Kontakt gebrachte Hautkontaktoberfläche aufweist;
eine flüssigkeitsundurchlässige Unterlage (20); und
einen Absorbierer (30), der zwischen der Oberlage und der Unterlage angeordnet ist, wobei:
der Absorbierer an einer Seite der Hautkontaktoberfläche mit einem Kompressionsbereich (50) in Kanalform versehen ist, wobei der Kompressionsbereich durch einen Kompressionsvorgang in Bezug auf mindestens die Oberlage und den Absorbierer eine erhöhte Dichte aufweist;
der Kompressionsbereich mit einem Hochkompressionsbereich (54A, 54B, 55A, 55B) versehen ist, der eine weiter erhöhte Dichte aufweist; **dadurch gekennzeichnet, dass** der Hochkompressionsbereich einen Kontaktabschnitt (54a, 54b, 55a, 55b) aufweist, der, in einer Draufsicht des absorbierenden Artikels, mit einem Nichtkompressionsbereich (30a), der keiner Kompression unterliegt, in Punktkontakt ist;
wobei
der Hochkompressionsbereich in der Draufsicht des absorbierenden Artikels viereckig geformt ist; und
der Kontaktabschnitt eine Ecke des Vierecks ist;
wobei:
in der Draufsicht des absorbierenden Artikels
der Kompressionsbereich (50) in einer Längsrichtung (L) des absorbierenden Artikels gebildet ist;
eine Vielzahl von Hochkompressionsbereichen (54A, 54B, 55A, 55B) in vorgegebenen Abständen in der Längsrichtung des absorbierenden Artikels gebildet sind;
der absorbierende Artikel weiter Folgendes umfasst:
eine erste Gruppe (G1) der Hochkompressionsbereiche, die die Kontaktabschnitte (54a, 55a) in Punktkontakt mit einer Endseite des Kompressionsbereichs in Kanalform aufweisen; und
eine zweite Gruppe (G2) der Hochkompressionsbereiche, die die Kontaktabschnitte (54b, 55b) in Punktkontakt mit der anderen Endseite des Kompressionsbereichs in Kanalform aufweisen.

2. Absorbierender Artikel gemäß Anspruch 1, wobei:
die erste Gruppe (G1) der Hochkompressionsbereiche (54A) und die zweite Gruppe (G2) der Hochkompressionsbereiche (54B) in einer versetzten Konfiguration arrangiert sind.

3. Absorbierender Artikel gemäß Anspruch 1, wobei:
der absorbierende Artikel weiter Folgendes umfasst:
eine dritte Gruppe (G3) von Hochkompressionsbereichen (55C), die zwischen der ersten Gruppe (G1) der Hochkompressionsbereiche (55A) und der zweiten Gruppe (G2) der Hochkompressionsbereiche (55B) gebildet sind; wobei die erste Gruppe (G1) der Hochkompressionsbereiche (55A) und die dritte Gruppe (G3) der Hochkompressionsbereiche (55C) in einer versetzten Konfiguration arrangiert sind; und
die zweite Gruppe (G2) der Hochkompressionsbereiche (55B) und die dritte Gruppe (G3) der Hochkompressionsbereiche (55C) in einer versetzten Konfiguration arrangiert sind.

4. Absorbierender Artikel gemäß Anspruch 1, der weiter Folgendes beinhaltet:
eine absorbierende Lage (21) zum Absorbieren von Fluid, wobei:
die absorbierende Lage zwischen dem Absorbierer (30) und der Unterlage (20) angeordnet ist; und
nur die Oberlage (10) an der Seite der Hautkontaktoberfläche des Absorbierers (20) angeordnet ist.

## Revendications

1. Un article absorbant (1) comprenant :
un feuillet supérieur perméable aux liquides (10) ayant une surface de contact avec la peau qui est amenée au contact de la peau d'une personne portant l'article ;
un feuillet arrière imperméable aux liquides (20) ; et
un absorbant (30) disposé entre le feuillet supérieur et le feuillet arrière, où :
l'absorbant est pourvu d'une région de compression (50) en forme de canal au niveau d'un côté de la surface de contact avec la peau, la région de compression ayant une densité accrue par rapport au moins au feuillet supérieur et à l'absorbant grâce à un processus de compression ;
la région de compression est pourvue d'une région de haute compression (54A, 54B, 55A, 55B) ayant une densité davantage accrue ; **caractérisé en ce que** la région de haute compression a une portion de contact (54a, 54b, 55a, 55b) en contact ponctuel avec une région de non compression (30a) non soumise à compression, dans une vue en plan de l'article absorbant ;
où
la région de haute compression est façonnée en un quadrilatère dans la vue en plan de l'article absorbant ; et
la portion de contact est un sommet du quadrilatère ;
où :
dans la vue en plan de l'article absorbant,
la région de compression (50) est formée dans une direction longitudinale (L) de l'article absorbant ;
une pluralité de régions de haute compression (54A, 54B, 55A, 55B) sont formées à intervalles prédéterminés dans la direction longitudinale de l'article absorbant ;
l'article absorbant inclut en sus :
un premier groupe (G1) des régions de haute compression ayant les portions de contact (54a, 55a) en contact ponctuel avec un côté d'extrémité de la région de compression en forme de canal ; et
un deuxième groupe (G2) des régions de haute compression ayant les portions de contact (54b, 55b) en contact ponctuel avec l'autre côté d'extrémité de la région de compression en forme de canal.

2. L'article absorbant selon la revendication 1, où :
le premier groupe (G1) des régions de haute compression (54A) et le deuxième groupe (G2) des régions de haute compression (54B) sont arrangés selon une configuration en quinconce.

3. L'article absorbant selon la revendication 1, où :
l'article absorbant inclut en sus :
un troisième groupe (G3) de régions de haute compression (55C) formé entre le premier groupe (G1) des régions de haute compression (55A) et le deuxième groupe (G2) des régions de haute compression (55B) ; où le premier groupe (G1) des régions de haute compression (55A) et le troisième groupe (G3) des régions de haute compression (55C) sont arrangés selon une configuration en quinconce ; et
le deuxième groupe (G2) des régions de haute compression (55B) et le troisième groupe (G3) des régions de haute compression (55C) sont arrangés selon une configuration en quinconce.

4. L'article absorbant selon la revendication 1 comprenant en sus :
un feuillet absorbant (21) pour absorber du fluide, où :
le feuillet absorbant est disposé entre l'absorbant (30) et le feuillet arrière (20) ; et
seul le feuillet supérieur (10) est disposé au niveau du côté de la surface de contact avec la peau de l'absorbant (20).
